# FASCICULE DE BREVET EUROPEEN

(11) **EP 0 984 911 B1**
(45) Date de publication et mention de la délivrance du brevet: **14.04.2004**
(21) Numéro de dépôt: 98925516.1
(22) Date de dépôt: 28.04.1998
(51) Int. Cl.: C07C 17/278, C07C 17/275, C07C 19/01, B01J 31/02

(54) **PROCEDE DE PREPARATION D'HYDROCARBURES HALOGENES**
VERFAHREN ZUR HERSTELLUNG VON HALOGINIERTEN KOHLENWASSERSTOFFEN
METHOD FOR PREPARING HALOGENATED HYDROCARBONS

(30) Priorité: 05.05.1997 BE 9700399; 08.08.1997 BE 9700669; 24.02.1998 BE 9800141
(43) Date de publication de la demande: 15.03.2000
(73) Titulaire: SOLVAY (Société Anonyme), 1050 Bruxelles (BE)
(72) Inventeur: MATHIEU, Véronique, B-1300 Wavre (BE); JANSSENS, Francine, B-1800 Vilvoorde (BE)
(74) Mandataire: Jacques, Philippe
(86) Numéro de dépôt international: PCT/EP1998/002586
(87) Numéro de publication internationale: WO 1998/050330

(56) Documents cités:
- EP-A- 0 729 932
- EP-A- 0 787 707
- WO-A-95/04022
- WO-A-97/05089
- WO-A-97/07083
- WO-A-97/15540
- FR-A- 1 288 511
- US-A- 3 651 019
- US-A- 3 862 978
- US-A- 5 446 217

## Description

La présente invention concerne un procédé de préparation d'hydrocarbures halogénés comprenant au moins 3 atomes de carbone par réaction catalytique entre un haloalcane et une oléfine.

L'addition d'un haloalcane sur une oléfine est une réaction bien connue. Cependant, il est parfois difficile de contrôler la réaction de telle sorte qu'une seule molécule d'oléfine s'additionne à une molécule d'haloalcane (formation d'un produit d'addition ou adduit 1:1).

Très souvent, des dérivés du cuivre sont utilisés pour catalyser cette réaction d'addition. Par exemple, M. Assener et D. Vofsi (J. Chem. Soc. 1887-1896, 1963) décrivent l'addition de tétrachlorure de carbone à des oléfines en présence de catalyseurs contenant du cuivre ou du fer. Toutefois, ce procédé présente l'inconvénient de nécessiter de longues périodes de chauffage pour obtenir le produit d'addition avec un rendement acceptable.

La demande de brevet WO 97/07083 décrit un procédé de préparation d'hydrocarbures halogénés sous l'action catalytique du chlorure cuivreux en présence de t.butylamine comme cocatalyseur. Dans un tel procédé, le rendement en produit de télomérisation est toutefois assez faible.

L'invention vise dès lors à fournir un procédé qui permet d'accéder avec d'excellents rendements aux hydrocarbures halogénés comprenant au moins 3 atomes de carbone, en une seule étape et au départ de réactifs facilement accessibles.

En conséquence, la présente invention concerne un procédé de préparation d'hydrocarbures halogénés comprenant au moins 3 atomes de carbone par réaction entre un haloalcane et une oléfine halogénée, caractérisé en ce qu'on effectue la réaction en présence
(a) d'un composé organique du cuivre, ledit composé organique étant choisi parmi les composés formés avec un composé organique acide choisi parmi les acides carboxyliques, l'acétylacétone, l'acétoacétate d'éthyle, le nitrométhane le diphénylméthane, la diméthylsulfone et leurs dérivés chlorés ou fluorés ;
(b) d'un cocatalyseur choisi parmi les amines aliphatiques comprenant de 3 à 25 atomes de carbone, les amides et les oxydes de trialkylphosphines ;
(c) éventuellement d'un solvant polaire.
Constituent typiquement des composés organiques acides, les acides carboxyliques tels que les acides formique, acétique, propionique, butyrique, acétylacétique, cyclohexanebutyrique, benzoïque. Conviennent également les acides carboxyliques chlorés ou fluorés, tels que les acides trichloroacétique et trifluoroacétique. D'autres composés organiques acides sont les composés possédant un atome d'hydrogène voisin d'un ou de plusieurs groupements électrocapteurs tels que les groupements carbonyle (C=O), sulfone (SO₂R), nitro (NO₂), phényle, choisis parmi l'acétylacétone, la trifluoroacétylacétone, la 1,1,1,5,5,5-hexafluoropentane-2,4-dione, l'acétoacétate d'éthyle, le nitrométhane, le diphénylméthane et la diméthylsulfone et leurs dérivés chlorés ou fluorés. Les composés de cuivre formés avec des composés organiques acides tels que ceux mentionnés ci-dessus sont utilisables dans le procédé selon la présente invention. Sont préférés les composés de cuivre formés avec des composés tels que l'acétylacétone, l'acétoacétate d'éthyle, l'acide acétique, l'acide cyclohexanebutyrique ainsi que leurs dérivés chlorés et fluorés. Les composés de cuivre (II) sont particulièrement préférés. Avantageusement, le catalyseur dans le procédé selon la présente invention est choisi parmi l'acétate de cuivre (II), le cyclohexanebutyrate de cuivre (II) et l'acétylacétonate de cuivre (II). Une préférence toute particulière est marquée pour le composé formé entre le cuivre (II) et l'acétylacétone (acétylacétonate de cuivre (II) abrévié par Cu(acac)₂) comme catalyseur dans le procédé selon la présente invention.

Dans un premier mode de mise en oeuvre du procédé selon l'invention, la réaction est réalisée en présence d'un solvant. Tout solvant dans lequel les réactifs forment le produit recherché avec un rendement satisfaisant peut être utilisé. Avantageusement, le solvant de la réaction est un alcool, un nitrile, un amide, une lactone, un oxyde de trialkylphosphine ou un autre solvant polaire. Les solvants oxygénés (c'est-à-dire dont la molécule contient au moins un atome d'oxygène) sont préférés.

Parmi les alcools utilisables comme solvant pour la réaction, on trouve notamment le méthanol, l'éthanol, l'isopropanol et le tert-butanol. Parmi les nitriles utilisables comme solvant pour la réaction, on trouve notamment des nitriles aliphatiques, notamment l'acétonitrile, le propionitrile, ou l'adiponitrile et des nitriles aromatiques, notamment le benzonitrile ou le tolunitrile. Parmi les nitriles, le propionitrile et l'adiponitrile sont préférés. Parmi les amides utilisables comme solvant pour la réaction, on trouve des amides linéaires comme le N,N-diméthylacétamide et le N,N-diméthylformamide et des amides cycliques comme la N-méthylpyrrolidone. On peut aussi mentionner l'hexaméthylphosphoramide. Parmi les lactones utilisables comme solvant pour la réaction, on peut citer notamment la γ-butyrolactone. Parmi les oxydes de trialkylphosphine utilisables comme solvant pour la réaction, on peut citer notamment les composés de formule (R₁R₂R₃)PO, dans laquelle R₁, R₂ et R₃ représentent des groupements alkyles en C3-C10, identiques ou différents, de préférence linéaires. On retient en particulier l'oxyde de tri-(n-butyl)phosphine, l'oxyde de tri-(n-hexyl)phosphine, l'oxyde de tri-(n-octyl)phosphine, l'oxyde de n-octyl-di-(n-hexyl)-phosphine, l'oxyde de n-hexyl-di-(n-octyl)-phosphine oxyde et leurs mélanges. Comme autres solvants polaires, on peut encore mentionner la 1,3-diméthyl-2-imidazolidinone, le diméthylsulfoxyde et le tétrahydrofuranne. De préférence, le solvant est un amide ou un oxyde de trialkylphosphine. De bons résultats ont en particulier été obtenus avec la N-méthylpyrrolidone, avec le N,N-diméthylacétamide, ainsi qu'avec un mélange d'oxyde de tri-(n-hexyl)phosphine, d'oxyde de tri-(n-octyl)phosphine, d'oxyde de n-octyl-di-(n-hexyl)-phosphine et d'oxyde de n-hexyl-di-(n-octyl)-phosphine.

Dans le procédé selon l'invention, on réalise la réaction en présence d'une amine aliphatique comprenant de 3 à 25 atomes de carbone, d'un amide ou d'un oxyde de trialkylphosphine comme cocatalyseur. Comme amides utilisables comme cocatalyseur, on peut citer la N-méthylpyrrolidone et le N,N-diméthylformamide. Comme oxydes de trialkylphosphine utilisables comme cocatalyseur, on peut citer les mêmes composés que ceux utilisables comme solvant dans le premier mode de réalisation de l'invention. On préfère utiliser une amine comme cocatalyseur, en particulier une amine primaire. Sont particulièrement préférées les amines aliphatiques comprenant de 3 à 22 atomes de carbone. Comme amines aliphatiques primaires utilisables dans le procédé selon l'invention, on peut citer la n.propylamine, l'isopropylamine, la n.butylamine, l'isobutylamine, la t.butylamine, la pentylamine et l'isoamylamine. Parmi ces amines, une préférence toute particulière est accordée aux amines dont la chaîne alkyle est ramifiée et plus spécialement aux amines tert-alkyle répondant la formule générale (I) 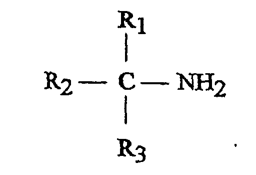 dans laquelle R₁, R₂ et R₃ représentent des groupements alkyle en C1-C8. Des amines répondant à la formule (I) sont notamment la t.butylamine et les amines tert-alkyles PRIMENE® 81-R et JM-T commercialisées par Rohm and Haas Company. La t.butylamine est tout particulièrement préférée.

Le système catalyseur-cocatalyseur préféré selon la présente invention est le système constitué d'un composé de cuivre (II) formé avec un composé organique acide et d'une amine primaire dont l'atome de carbone voisin du groupe NH₂ est un atome de carbone quaternaire, c'est-à-dire dépourvu d'atome d'hydrogène. Est particulièrement préféré, le système catalyseur-cocatalyseur formé par l'acétylacétonate de cuivre (II) et la t.butylamine.

Les haloalcanes engagés dans le procédé selon la présente invention sont en général des composés organiques saturés. Ils possèdent de préférence de un à trois atomes de carbone et de préférence au moins 2 atomes de chlore. Ils peuvent également comprendre d'autres substituants tels que d'autres atomes d'halogène, des groupes alkyle ou halogénoalkyle. A titre d'exemples d'haloalcanes selon la présente invention, on peut citer le dichlorométhane, le chloroforme, le tétrachlorure de carbone, le 1,1,1-trichloroéthane, le 1,1,1-triehloro-2,2,2-trifluoroéthane. Le tétrachlorure de carbone est tout particulièrement préféré.

L'oléfine engagée dans le procédé selon la présente invention est en général l'éthylène, le propylène ou un butène, eux-mêmes étant éventuellement substitués par des atomes d'halogène, des groupes alkyle ou halogénoalkyle, des groupes nitrile (CN) ou acide carboxylique (COOH). Les oléfines halogénées conviennent particulièrement bien. A titre d'exemples non limitatifs d'oléfines halogénées on peut citer le chlorure de vinyle, le chlorure de vinylidène, le trichloréthylène, les divers isomères des chloropropènes comme le 1-chloroprop-1-ène, le 2-chloroprop-1-ène et le 3-chloroprop-1-ène. D'excellents résultats ont été obtenus avec le chlorure de vinyle et le 2-chloroprop-1-ène.

Les hydrocarbures halogénés obtenus selon le procédé de la présente invention appartiennent, en général, à la famille des chloropropanes, des chlorobutanes ou des chloropentanes. Les atomes de carbone desdits chloropropanes, chlorobutanes et chloropentanes peuvent également être substitués par d'autres groupes fonctionnels tels que d'autres atomes d'halogène (comme des atomes de brome ou d'iode), des groupes alkyle ou halogénoalkyle, des groupes nitrile (CN) ou acide carboxylique (COOH). Les chloropropanes et les chlorobutanes non substitués par d'autres groupes fonctionnels sont préférés.

De préférence, les hydrocarbures halogénés obtenus selon le procédé de la présente invention répondent à la formule générale CnH₍₂ₙ₊₂₎₋ₚClₚ dans laquelle n est un nombre entier et possède les valeurs 3 ou 4, p est un nombre entier qui possède les valeurs 3 à 7. Des exemples de composés obtenus selon le procédé de la présente invention sont le 1,1,1,3,3-pentachloropropane, le 1,1,1,3,3-pentachlorobutane, le 1,1,1,3-tétrachloropropane, le 1,1,3,3-tétrachlorobutane, le 1,1,1,3,3,3-hexachlororopropane et le 1,1-dichloro-2-trichlorométhylpropane. Parmi ces composés, le 1,1,1,3,3-pentachloropropane, le 1,1,1,3,3-pentachlorobutane et le 1,1-dichloro-2-trichlorométhylpropane sont préférés. Le 1,1,1,3,3-pentachlorobutane et le 1,1,1,3,3-pentachloropropane sont tout particulièrement préférés.

Le rapport molaire entre le catalyseur et l'oléfine est habituellement supérieur ou égal à 0,0001. Avantageusement, il est supérieur ou égal à 0,001. De préférence, il est supérieur ou égal à 0,005. Le rapport molaire entre le catalyseur et l'oléfine est habituellement inférieur ou égal à 1. Avantageusement, il est inférieur ou égal à 0,5. De préférence, il est inférieur ou égal à 0,1.

La quantité de solvant engagé dans le premier mode de mise en oeuvre du procédé selon l'invention n'est pas critique. Toutefois, une solution trop diluée n'est pas favorable à un rendement et à un taux de conversion élevés. De préférence, le rapport molaire du solvant à l'oléfine est égal ou supérieur à 0,05. Avantageusement, ce rapport est égal ou supérieur à 0,1. Le rapport molaire du solvant à l'oléfine est en général égal ou inférieur à 30. Avantageusement, il est égal ou inférieur à 20. D'une manière préférée, ce rapport est égal ou supérieur à 0,2 et égal ou inférieur à 15. D'une manière toute préférée, il est égal ou supérieur à 1 et égal ou inférieur à 10. Dans le milieu réactionnel, la quantité de solvant peut varier, sur une base molaire, d'environ 5 à environ 500 fois la quantité de catalyseur, de préférence d'environ 10 à environ 200 fois.

Dans le procédé selon l'invention, le rapport molaire entre le cocatalyseur et l'oléfine est généralement supérieur ou égal à 0,01. De préférence, ce rapport molaire est supérieur ou égal à 0,05. Avantageusement, ce rapport molaire est supérieur ou égal à 0,1. Toutefois, ce rapport molaire est habituellement inférieur ou égal à 2. De préférence, ce rapport molaire est inférieur ou égal à 1. Avantageusement, ce rapport molaire est inférieur ou égal à 0,5. La quantité de cocatalyseur mise en oeuvre peut varier, sur une base molaire, d'environ 0,1 à environ 25 fois la quantité de catalyseur, de préférence d'environ 0,5 à environ 20 fois.

Le procédé selon l'invention peut être réalisé en continu ou en discontinu.

Il est entendu que la quantité de catalyseur, de solvant ou de cocatalyseur mise en oeuvre est exprimée, dans un procédé en discontinu, par rapport à la quantité initiale d'oléfine mise en oeuvre et, dans un procédé en continu, par rapport à la quantité stationnaire d'oléfine présente dans le réacteur.

Le rapport molaire entre l'haloalcane et l'oléfine mise en oeuvre peut varier dans de larges mesures. Ce rapport est en général égal ou supérieur à 0,1. Avantageusement, ce rapport est égal ou supérieur à 0,5. D'une manière préférée, il est supérieur ou égal à 1. D'excellents résultats sont obtenus lorsque ce rapport est d'au moins 1,2. On a en effet observé de manière surprenante qu'il est possible, dans le procédé selon l'invention, de travailler avec un rapport entre l'haloalcane et l'oléfine proche de la stoechiométrie sans affecter de manière sensible la sélectivité. Généralement, ce rapport est égal ou inférieur à 15. Avantageusement, ce rapport est égal ou inférieur à 10. D'une manière préférée, ce rapport est égal ou inférieur à 5.

Généralement, la réaction se déroule à une température supérieure ou égale à la température ambiante. De préférence, la température est supérieure ou égale à 50 °C. Avantageusement, la température est supérieure ou égale à 70 °C. Toutefois, la température est généralement inférieure ou égale à 200 °C. De préférence, la température est inférieure ou égale à 175 °C. Avantageusement, la température est inférieure ou égale à 150 °C. Une préférence toute particulière est montrée pour une température inférieure ou égale à 100 °C.

La durée de la réaction dans un procédé en discontinu ou le temps de séjour dans un procédé en continu sont fonction de divers paramètres tels que la température de réaction, la concentration en réactifs et en catalyseur dans le mélange réactionnel et leurs rapports molaires. En général, en fonction de ces paramètres, le temps de séjour ou la durée de réaction peuvent varier de 5 minutes à 10 heures. Avantageusement, dans un procédé en discontinu, le temps de réaction est généralement supérieur ou égal à 30 minutes avec une préférence pour les temps de réaction supérieurs ou égaux à 60 minutes. Toutefois, le temps de réaction est habituellement inférieur ou égal à 10 heures avec une préférence pour les temps de réaction inférieurs ou égaux à 8 heures.

La pression est généralement choisie de façon à maintenir le milieu réactionnel en phase liquide. La pression mise en oeuvre varie en fonction de la température du milieu réactionnel. La pression est habituellement supérieure ou égale à la pression atmosphérique et inférieure ou égale à 10 bars.

Dans le procédé selon l'invention, la présence d'un cocatalyseur permet généralement de réaliser la réaction en absence de solvant, le système catalyseur/cocatalyseur étant le plus souvent soluble dans l'haloalcane mis en oeuvre. Toutefois, le procédé selon l'invention peut aussi être réalisé à la fois en présence d'un solvant, conformément au premier mode de réalisation de l'invention et en présence d'un cocatalyseur, conformément au deuxième mode de réalisation de l'invention.

Les hydrocarbures halogénés obtenus selon le procédé de l'invention sont des précurseurs des analogues fluorés correspondants, lesquels peuvent être facilement obtenus par traitement avec du fluorure d'hydrogène en présence d'un catalyseur tel qu'un sel d'antimoine, un sel de titane, un sel de tantale ou un sel d'étain.

Les exemples ci-après illustrent l'invention de manière non limitative.

### Exemples 1-13

On a préparé du 1,1,1,3,3-pentachloropropane au départ de chlorure de vinyle (VC) et de tétrachlorure de carbone ou du 1,1,1,3,3-pentachlorobutane au départ de 2-chloroprop-1-ène (2CPe) et de tétrachlorure de carbone, par réaction entre ces réactifs en présence d'un composé organique du cuivre et d'une amine. Pour ce faire, on a introduit les réactifs, le catalyseur et le cocatalyseur dans un autoclave de 300 ml dont les parois internes sont recouvertes de téflon. L'appareil a ensuite été fermé hermétiquement, placé dans un four vertical et la température a été augmentée progressivement et maintenue à 90 °C pendant la durée de la réaction. L'agitation a été assurée par un barreau magnétique placé dans le fond de l'autoclave. En fin de réaction, on a laissé refroidir l'autoclave, un échantillon de liquide a été prélevé à la seringue et dosé par une méthode chromatographique pour déterminer le taux de conversion de l'oléfine et la sélectivité en hydrocarbure halogéné. Les résultats obtenus sont rassemblés au tableau I.

### Exemple 14 (comparatif)

On a répété l'exemple 12 en remplaçant l'acétylacétonate de cuivre (II) par du CuCl. Après 2 heures de réaction, la conversion du 2-chloroprop-1-ène ne dépassait pas 60 %.

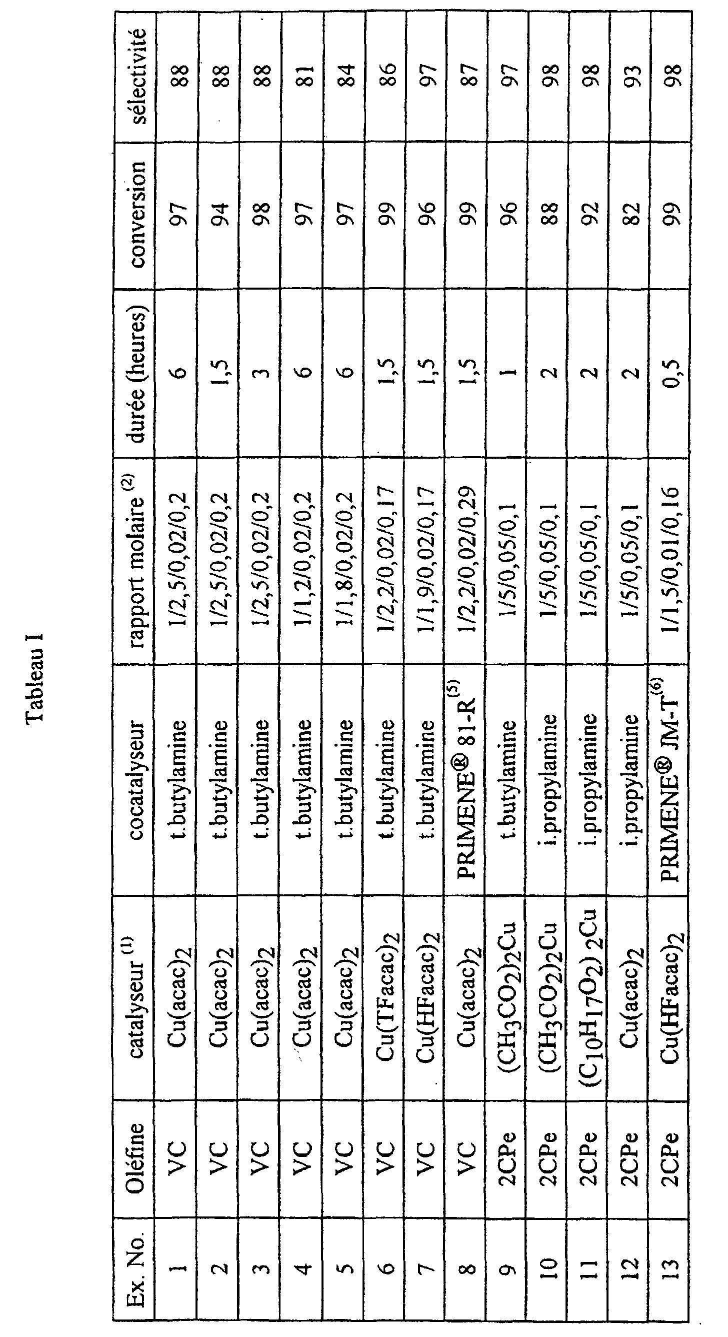

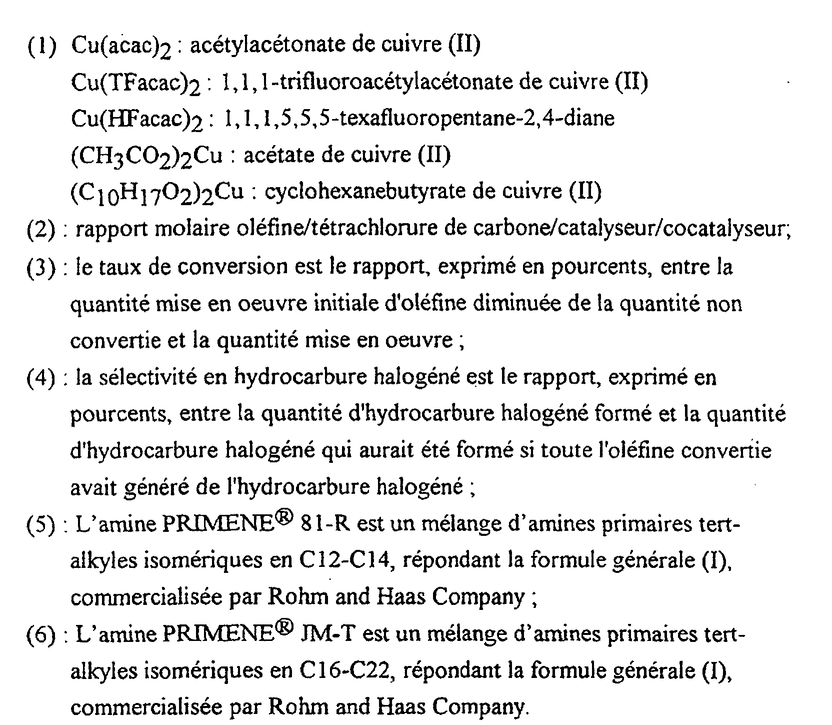

### Exemples 15-18

On a préparé du 1,1,1,3,3-pentachlorobutane au départ de 2-chloroprop-1-ène et de tétrachlorure de carbone en présence de différents solvants et d'acétylacétonate de cuivre (II) à titre de catalyseur. La durée de réaction était de 2 heures. Les rapports molaires des réactifs, les températures de réaction et les résultats obtenus sont rassemblés dans le tableau II.

### Exemples 19-20

On a préparé du 1,1,1,3,3-pentachlorobutane au départ de 2-chloroprop-1-ène et de tétrachlorure de carbone en présence d'acétylacétonate de cuivre (II) à titre de catalyseur et d'un mélange de 4 trialkylphosphine oxydes (tri-(n-hexyl)phosphine oxyde, tri-(n-octyl)phosphine oxyde, n-octyl-di-(n-hexyl)-phosphine oxyde et n-hexyl-di-(n-octyl)-phosphine oxyde), commercialisé par CYTEC sous la dénomination CYANEX® 923. La durée de réaction était de 2 heures. Les rapports molaires des réactifs, les températures de réaction et les résultats obtenus sont également présentés dans le tableau II.

**Tableau II**

| Ex. | Solvant | Rapport molaire 2-CPe / CCl₄ / Cu(acac)₂ / solvant | Température | Conversion | Sélectivité |
|---|---|---|---|---|---|
| 15 | N-méthylpyrrolidone | 1 / 2 / 0,06 / 3,8 | 100 °C | 96 | 97 |
| 16 | N,N-diméthylacétamide | 1 / 3,5 / 0,06 / 4,1 | 90 °C | 67 | 99 |
| 17 | 1,3-diméthyl-2-imidazolidinone | 1 / 2,2 / 0,05 / 3,2 | 90 °C | 46 | 97 |
| 18 | N,N-diméthylformamide | 1 / 1,9 / 0,06 / 5,7 | 100 °C | 72 | 96 |
| 19 | CYANEX® 923 | 1 / 1,8 / 0,047 / 0,99 | 90 °C | 86 | 95 |
| 20 | CYANEX® 923 | 1 / 5 / 0,05 / 0,24 | 90 °C | 88 | 95 |

## Revendications

1. Procédé de préparation d'hydrocarbures halogénés comprenant au moins 3 atomes de carbone par réaction entre un haloalcane et une oléfine halogénée, **caractérisé en ce qu'**on effectue la réaction en présence
(a) d'un composé organique du cuivre, ledit composé organique étant choisi parmi les composés formés avec un composé organique acide choisi parmi les acides carboxyliques, l'acétylacétone, l'acétoacétate d'éthyle, le nitrométhane le diphénylméthane, la diméthylsulfone et leurs dérivés chlorés ou fluorés ;
(b) d'un cocatalyseur choisi parmi les amines aliphatiques comprenant de 3 à 25 atomes de carbone, les amides et les oxydes de trialkylphosphines ;
(c) éventuellement d'un solvant polaire.

2. Procédé selon la revendication 1 dans lequel le composé organique acide est un acide carboxylique, choisi parmi l'acide acétique, l'acide cyclohexanebutyrique et leurs dérivés chlorés ou fluorés.

3. Procédé selon la revendication 1 dans lequel le composé organique acide est choisi parmi l'acétylacétone, l'acétoacétate d'éthyle, et leurs dérivés chlorés ou fluorés.

4. Procédé selon l'une quelconque des revendications 1 à 3 dans lequel le composé organique de cuivre utilisé comme catalyseur se trouve à l'étage d'oxydation (II).

5. Procédé selon la revendication 3 ou 4 dans lequel le catalyseur est l'acétylacétonate de cuivre (II).

6. Procédé selon l'une quelconque des revendications 1 à 5 dans lequel on effectue la réaction en présence d'un solvant polaire choisi parmi un alcool, un nitrile, un amide, une lactone ou un oxyde de trialkylphosphine.

7. Procédé selon l'une quelconque des revendications 1 à 6 dans lequel on effectue la réaction en présence d'un solvant polaire oxygéné.

8. Procédé selon l'une quelconque des revendications 1 à 7 dans lequel l'amine est une amine tert-alkyle répondant la formule générale; 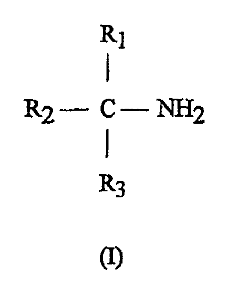 dans laquelle R₁, R₂ et R₃ représentent des groupements alkyle en C1-C8.

9. Procédé selon l'une quelconque des revendications 1 à 8 dans lequel l'hydrocarbure halogéné préparé est le 1,1,1,3,3-pentachlorobutane ou le 1,1,1,3,3-pentachloropropane.

10. Procédé selon la revendication 9, dans lequel l'hydrocarbure halogéné préparé est le 1,1,1,3,3-pentachlorobutane.

11. Procédé de fabrication d'hydrocarbures fluorés comprenant au moins 3 atomes de carbone, selon lequel
(a) on prépare des hydrocarbures halogénés comprenant au moins 3 atomes de carbone selon le procédé de l'une quelconque des revendications 1 à 10 ; et
(b) on soumet les hydrocarbures halogénés obtenus à un traitement avec du fluorure d'hydrogène en présence d'un catalyseur.

## Patentansprüche

1. Verfahren zur Herstellung von wenigstens drei Kohlenstoffatome aufweisenden halogenierten Kohlenwasserstoffen durch Umsetzung zwischen einem Halogenalkan und einem halogenierten Olefin, **dadurch gekennzeichnet, daß** die Umsetzung in Gegenwart
(a) einer organischen Kupferverbindung, wobei die organische Verbindung unter den Verbindungen ausgewählt ist, die mit einer sauren organischen Verbindung gebildet werden, ausgewählt unter Carbonsäuren, Acetylaceton, Ethylacetoacetat, Nitromethan, Diphenylmethan, Dimethylsulfon und deren chlorierten oder fluorierten Derivaten;
(b) eines Cokatalysators, ausgewählt unter aliphatischen Aminen mit 3 bis 25 Kohlenstoffatomen, Amiden und Trialkylphosphinoxiden;
(c) gegebenenfalls eines polaren Lösungsmittels
vorgenommen wird.

2. Verfahren nach Anspruch 1, worin die saure organische Verbindung eine unter Essigsäure, Cyclohexanbuttersäure und deren chlorierten oder fluorierten Derivaten ausgewählte Carbonsäure ist.

3. Verfahren nach Anspruch 1, worin die saure organische Verbindung unter Acetylaceton, Ethylacetoacetat und deren chlorierten oder fluorierten Derivaten ausgewählt ist.

4. Verfahren nach einem der Ansprüche 1 bis 3, worin die als Katalysator verwendete organische Kupferverbindung im Oxidationszustand (II) vorliegt.

5. Verfahren nach Anspruch 3 oder 4, worin der Katalysator das Kupfer(II)acetylacetonat ist.

6. Verfahren nach einem der Ansprüche 1 bis 5, worin die Umsetzung in Gegenwart eines unter einem Alkohol, einem Nitril, einem Amid, einem Lacton oder einem Trialkylphosphinoxid ausgewählten polaren Lösungsmittels vorgenommen wird.

7. Verfahren nach einem der Ansprüche 1 bis 6, worin die Umsetzung in Gegenwart eines oxygenierten polaren Lösungsmittels ausgeführt wird.

8. Verfahren nach einem der Ansprüche 1 bis 7, worin das Amin ein tert.-Alkylamin entsprechend der allgemeinen Formel: 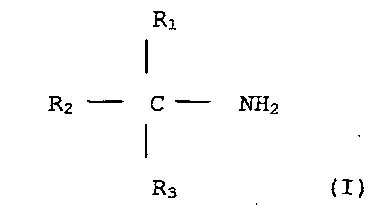 ist, worin R₁, R₂ und R₃ für C₁-C₈-Alkylgruppen stehen.

9. Verfahren nach einem der Ansprüche 1 bis 8, worin der hergestellte Halogenkohlenwasserstoff das 1,1,1,3,3-Pentachlorbutan oder das 1,1,1,3,3-Pentachlorpropan ist.

10. Verfahren nach Anspruch 9, worin der hergestellte Halogenkohlenwasserstoff das 1,1,1,3,3-Pentachlorbutan ist.

11. Verfahren zur Herstellung von wenigstens drei Kohlenstoffatome aufweisenden Fluorkohlenwasserstoffen, wonach
(a) wenigstens drei Kohlenstoffatome aufweisende halogenierte Kohlenwasserstoffe nach dem Verfahren gemäß einem der Ansprüche 1 bis 10 hergestellt werden; und
(b) die erhaltenen halogenierten Kohlenwasserstoffe in Gegenwart eines Katalysators einer Behandlung mit Fluorwasserstoff unterworfen werden.

## Claims

1. Process for the preparation of halohydrocarbons comprising at least 3 carbon atoms, by reaction between a haloalkane and a halogenated olefin, in which the reaction is carried out in the presence of
(a) an organocopper compound as catalyst, which organocopper selected from compounds formed with an organic acid compound selected from carboxylic acids, acetylacetone, ethyl acetoacetate, nitromethane, diphenylmethane, dimethyl sulphone, and chloro or fluoro derivatives thereof, and
(b) a cocatalyst chosen from amines comprising from 3 to 25 carbon atoms, amides and trialkylphosphine oxides.
(c) optionally a polar solvent.

2. Process according to claim 1, wherein the organic acid compound is a carboxylic acid chose from acetic acid, cyclohexanebutyric acid and their chloro or fluoro devivates.

3. Process according to claim 1, wherein the organic acid compound is selected from acetylacetone, ethyl acetoacetate and their chloro or fluoro derivates.

4. Process according to any one of Claims 1 to 3, in which the organocopper compound used as catalyst is in the oxidation state (II).

5. Process according to Claim 3 or 4, in which the catalyst is copper (II) acetylacetonate.

6. Process according to any one of Claims 1 to 5, in which the reaction is carried out in the presence of a polar solvent selected from an alcohol, a nitrile, an amide, a lactone or a trialkylphosphine oxide.

7. Process according to any one of Claims 1 to 6, in which the reaction is carried out in the presence of an oxygenated polar solvent

8. Process according to any one of Claims 1 to 7, in which the amine is a tert-alkylamine corresponding to the general formula 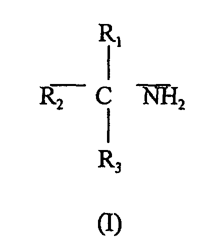 in which R₁, R₂ and R₃ represent C1-C8 alkyl groups.

9. Process according to any one of Claims 1 to 8, in which the halohydrocarbon prepared is 1,1,1,3,3-pentachlorobutane or 1,1,1,3,3-pentachloropropane.

10. Process according to Claim 9 in which the halohydrocarbon prepared is 1,1,1,3,3-pentachlorobutane.

11. Process for the manufacture of fluorinated hydrocarbons comprising at least 3 carbon atoms, wherein,
(a) halogenated hydrocarbons comprising at least 3 carbons atoms are prepared according to the process of any one of Claims 1 to 10.
(b) the obtained halogenated hydrocarbons are submitted to a treatment with hydrogen fluoride in the presence of a catalyst.
